# DEMANDE DE BREVET EUROPEEN

(11) **EP 4 552 612 A1**
(43) Date de publication de la demande: **14.05.2025**
(21) Numéro de dépôt: 24211751.3
(22) Date de dépôt: 08.11.2024
(51) Int. Cl.: A61F 2/00, A61F 5/48, A61F 5/41

(54) **DISPOSITIF DE COMPRESSION PENIENNE**

(30) Priorité: 08.11.2023 FR 2312149
(71) Demandeur: Androdias, Hugo, 69007 Lyon (FR)
(72) Inventeur: Androdias, Hugo, 69007 Lyon (FR)
(74) Mandataire: Cabinet Nony

(57) **Abrégé**

L'invention concerne un dispositif de compression pénienne comportant :
- des branches supérieure (12s) et inférieure (12i), et
- des jonctions droite (14d) et gauche (14g) reliant des extrémités droites (16ds, 16di) des branches supérieure et inférieure, et des extrémités gauches (16gs, 16gi) des branches supérieure et inférieure, respectivement, de manière à délimiter une ouverture (20), au moins une des branches supérieure et inférieure, dite « branche pliable », étant pliable élastiquement par pincement du dispositif entre les jonctions droite et gauche, dispositif dans lequel ladite branche pliable est constituée en un ou plusieurs matériaux présentant une dureté Shore A inférieure à 40.

## Description

### Domaine technique

La présente invention est relative à un dispositif de compression du pénis destiné à serrer extérieurement le pénis pour empêcher les fuites urinaires, ainsi qu'un procédé associé.

### Art antérieur

Un dispositif de compression pénienne est adapté pour, dans une position de service, compresser extérieurement le pénis afin de fermer l'urètre sans bloquer la circulation sanguine.

Il peut comporter des première et deuxième branches dont les premières extrémités sont reliées par une charnière, et dont les deuxièmes extrémités sont reliées au moyen d'une attache désactivable. L'attache rend délicate la mise en position de service.

On connaît également des dispositifs de compression pénienne dans lesquels les deuxièmes extrémités des branches sont également reliées entre elles par une charnière. En particulier, le dispositif décrit dans WO2010042813 présente deux branches sous la forme de lames ressort plates, de sections transversales constantes, jointes à leurs premières et deuxièmes extrémités, par des première et deuxième jonctions, respectivement. Dans la position de repos, les branches délimitent ensemble une ouverture oblongue. En comprimant le dispositif latéralement de manière à rapprocher les première et deuxième jonctions l'une de l'autre (figure 10b de WO2010042813), la hauteur de l' ouverture peut être augmentée jusqu' à autoriser l'introduction du pénis dans l'ouverture. Le relâchement de cette compression produit un retour élastique vers la position de repos, et une compression du pénis.

La dureté du matériau constitutif des lames ressort limite la pression qu'elles peuvent exercer sans être traumatisantes. Le dispositif n'est donc pas fiable et une poche est prévue pour récupérer l'urine qui s'échappe. Par ailleurs, il n'est pas confortable, est encombrant et se rompt après quelques mois d'utilisation.

WO2019094707, ou US2018060012, décrit un dispositif monobloc annulaire, sans attache, en un matériau élastique. Ce dispositif permet la miction dans la position de service. Des anses supérieure et inférieure permettent de déformer le dispositif pour l'extraire de la position de service. Cette extraction mobilise les deux mains de l'utilisateur. Le dispositif est encombrant et peu confortable. Les anses compliquent le nettoyage et peuvent potentiellement se rompre avec le temps. Enfin, le dispositif est asymétrique, ce qui nécessite une attention particulière lors de la mise en position de service.

EP3167844 décrit un dispositif annulaire en silicone dont l'ouverture peut être déformée par écartement radial, par la conjonction d'une traction vers le haut et d'une traction vers le bas. Ce dispositif est difficile à mettre en position de service et à retirer. En particulier, ces opérations requièrent les deux mains de l'utilisateur. En outre, le dispositif est asymétrique, ce qui nécessite une attention particulière lors de la mise en position de service. Enfin, dans la position de service, il comprime excessivement les vaisseaux sanguins.

EP2695584 décrit un dispositif annulaire dont l'ouverture peut être déformée par pincement. La déformation élastique est obtenue au moyen d'une structure interne métallique. Cette structure métallique se rompt en quelques mois, en particulier au milieu des branches. Elle nuit également au confort dans la position de service. Le dispositif est en outre encombrant et coûteux à fabriquer. Enfin le dispositif comporte, en surface, des cavités qui sont préjudiciables à l'hygiène et exposent la structure interne métallique à l'environnement extérieur (eau, oxydation, bactéries, etc.).

Il existe un besoin permanent pour améliorer les dispositifs de l'art antérieur, en particulier pour améliorer le compromis entre :
- facilité de la mise en position de service et de l'extraction hors de la position de service ;
- efficacité de la compression de l'urètre ;
- encombrement ;
- durée de vie ;
- confort ;
- discrétion ;
- hygiène ;
- facilité de nettoyage, et
- coût de fabrication.

Un but de l'invention est de répondre à ce besoin.

### Résumé de l'invention

Selon l'invention, on atteint ce but avec un dispositif de compression pénienne comportant, de préférence constitué par :
- des branches supérieure et inférieure et
- des jonctions droite et gauche reliant les extrémités droites des branches supérieure et inférieure, et les extrémités gauches des branches supérieure et inférieure, respectivement, de manière à délimiter une ouverture,
au moins une des branches supérieure et inférieure, de préférence chacune des branches supérieure et inférieure, dite « branche pliable », étant pliable élastiquement par pincement du dispositif entre les jonctions droite et gauche.

**Selon un premier aspect principal de l'invention,** ladite branche pliable est constituée en un ou plusieurs matériaux présentant une dureté Shore A inférieure à 50.

L'utilisation d'un matériau mou pour fabriquer des branches nécessite une adaptation des branches afin que les branches soient pliables élastiquement, en particulier en augmentant l'épaisseur des branches. D'autres adaptations, connues de l'homme de l'art, peuvent être envisagées.

L'invention fournit ainsi un dispositif qui présente des branches dont la première fonction est similaire à celle des branches sous la forme de lames ressort plates décrites dans WO2010042813 : sous l'effet du pincement, les deux branches se plient pour développer l'ouverture qui les sépare et ainsi atteindre la position de montage autorisant l'introduction du pénis de l'utilisateur dans l'ouverture. Après cessation du pincement, les branches reviennent élastiquement vers leur position de repos, mais entrent en butée sur le pénis, dans la position de service.

Comme on le verra plus en détail dans la suite de la description, la faible dureté des branches permet au dispositif d'exercer une pression plus élevée dans la position de service, avec un meilleur confort. En outre, le dispositif présente une plus grande compacité et s'adapte mieux au corps de l'utilisateur.

**Selon un deuxième aspect principal de l'invention,** au moins une des jonctions droite et gauche, de préférence chaque jonction droite et gauche est déformable élastiquement sous l'effet du pincement de manière à écarter les extrémités de la branche supérieure et de la branche inférieure qui sont physiquement connectées à ladite jonction.

Comme on le verra plus en détail dans la suite de la description, l'ouverture peut être ainsi élargie, selon la direction de la hauteur, par déformation des jonctions, ce qui confère au dispositif une compacité élevée.

La déformation est élastique. Dans la position de service, l'élasticité des jonctions peut ainsi avantageusement contribuer à la pression exercée sur l'urètre.

De préférence, une jonction, de préférence chaque jonction présente la forme d'un C, débouchant vers l'ouverture, les branches étant reliées aux extrémités du C. Le pincement contribue ainsi à ouvrir les C des jonctions, c'est-à-dire à aplatir les jonctions.

Bien entendu, les caractéristiques, optionnelles ou non, des différents aspects principaux de l'invention peuvent être combinées, dans la mesure où elles sont techniquement compatibles.

Quel que soit l'aspect principal, le dispositif peut encore comporter une ou plusieurs des caractéristiques optionnelles suivantes :
- ladite branche pliable présente, sur un segment de ladite branche pliable représentant plus de 80% de la longueur de ladite branche pliable, un profil transversal présentant une aire supérieure à 150 mm² ;
- au moins une des jonctions droite et gauche, de préférence chaque jonction droite et gauche est déformable élastiquement sous l'effet du pincement de manière à écarter les extrémités de la branche supérieure et de la branche inférieure qui sont physiquement connectées à ladite jonction ;
- ladite jonction déformable élastiquement présente la forme d'un C débouchant vers l'ouverture, les branches étant reliées aux extrémités du C ;
- le dispositif est monobloc et de préférence constitué en un ou plusieurs élastomères ;
- plus de 90% la surface extérieure du dispositif est convexe ;
- le dispositif est configuré de manière que le rapport de la hauteur de l'ouverture dans la position de montage sur la hauteur de l'ouverture dans la position de repos est supérieur à 5 ;
- le dispositif est configuré de manière que l'ouverture présente la forme d'une fente dans la position de repos, et présente un contour arrondi le long des branches et un contour sensiblement rectiligne le long des jonctions dans une position de montage obtenue, à partir de la position de repos, par pincement du dispositif entre les jonctions droite et gauche ;
- l'ouverture est symétrique par rapport à un plan longitudinal médian et par rapport à un plan transversal médian ;
- le dispositif est constitué en un ou plusieurs matériaux suffisamment souple(s) et élastique(s) pour que, à partir de la position de repos, deux points à la périphérie du dispositif et à proximité des extrémités droite et gauche du dispositif, respectivement, puissent être rapprochés jusqu'à se toucher, sans déformation plastique ni rupture du dispositif

L'invention concerne un procédé de compression du pénis d'un utilisateur, le procédé comportant les étapes suivantes :
a) pincement d'un dispositif selon l'invention initialement dans la position de repos, entre les jonctions droite et gauche, par l'utilisateur, de préférence avec une seule main, jusqu'à obtention de la position de montage ;
b) introduction du pénis dans l'ouverture du dispositif ;
c) relâchement du dispositif, de manière qu'il atteigne, par retour élastique vers la position de repos, la position de service.

### Brève description des figures

D'autres caractéristiques et avantages de l'invention apparaitront encore à l'examen de la description qui va suivre et au regard du dessin annexé dans lequel :
- la figure 1 [Fig 1] représente un dispositif selon l'invention dans la position de repos, en vues de face (1A), de dessus (1B), de droite (1C) et en coupe transversale A-A (1D);
- la figure 2 [Fig 2] représente, en perspective, dans la position de repos, le dispositif de la figure 1 ;
- la figure 3 [Fig 3] représente, vu de face, le dispositif de la figure 1 dans la position de montage;
- la figure 4 [Fig 4] représente, en perspective, le dispositif de la figure 1 dans la position de service ; et
- la figure 5 [Fig 5] représente un dispositif selon l'invention vu de dessus, dans une position dans laquelle les extrémités du dispositif se touchent.

Dans les différentes figures, des références identiques sont utilisées pour désigner des organes identiques ou similaires.

### Définitions

Une « branche » est classiquement un organe allongé suivant un axe principal, dont la longueur, mesurée selon ledit axe principal, est supérieure à 1,5 fois, de préférence supérieure à deux fois, la plus grande dimension transversale, c'est-à-dire mesurée dans une section transversale, en considérant toutes les sections transversales le long de la branche. La section transversale peut être constante ou non le long de l'axe principal. Une branche peut être rectiligne ou non. Une tige est un exemple de branche.

Les deux branches sont connectées par une jonction qui, comme la branche, délimite partiellement le contour de l'ouverture. On considère que la jonction commence lorsque la courbure de ce contour augmente fortement. La limite qui sépare la j onction et l'extrémité d'une branche connectée à la jonction peut être positionnée, sur le contour, à l'endroit où le centre de courbure du contour est du côté de l'ouverture et où le rayon de courbure devient inférieur à 1 cm lorsqu'on se rapproche de l'extrémité du dispositif la plus proche de la jonction.

La « position de service » est la position dans laquelle le dispositif est porté sans être tenu à la main par l'utilisateur, le pénis de l'utilisateur étant ceinturé par le dispositif, les branches pressant l'urètre de manière à empêcher toute fuite d'urine. Dans la position de service, le contact du pénis avec le dispositif ne s'étend de préférence pas sur tout le périmètre, ou « contour », de l'ouverture. En particulier, il ne s'étend de préférence pas sur les portions droite et gauche du contour de l'ouverture, dans les régions des jonctions. Le dispositif est de préférence configuré de manière que, dans la position de service, il n'exerce une pression sur le pénis que dans le plan de l'ouverture.

La « position de repos » est la position dans laquelle le dispositif n'est soumis à aucune force extérieure tendant à élargir l'ouverture. Une pièce dans la position de repos est dite « au repos ». De préférence, l'ouverture présente une aire minimale dans cette position.

La « position de montage » est la position dans laquelle le dispositif est pincé de manière que l'ouverture présente une aire suffisante pour que le pénis de l'utilisateur puisse être introduit dans l'ouverture. De préférence, l'ouverture présente une aire maximale dans cette position.

« L'utilisateur » est la personne qui, dans la position de service, porte le dispositif.

Les adjectifs « supérieur », « inférieur », « droit », « gauche », « vertical » et « horizontal » sont utilisés, à des fins de clarté, en référence à une position de service dans laquelle le dispositif est utilisé « horizontalement », c'est-à-dire les branches supérieure et inférieure étant sensiblement horizontales, la droite et la gauche étant déterminées du point de vue de l'utilisateur. La « hauteur » de l'ouverture, mesurée selon la direction verticale, est également définie en référence à ladite position de service.

Sauf indication contraire, la « longueur » d'un organe est mesurée en suivant l'axe principal de l'organe, par exemple en mesurant la distance entre les deux extrémités d'une branche en suivant la courbure éventuelle de la branche.

Un plan « transversal » est un plan perpendiculaire à l'axe principal d'un organe. Une section transversale est une section dans un plan de coupe transversale.

Un plan longitudinal est un plan qui inclut l'axe principal d'un organe.

L'axe X de l'ouverture est la droite perpendiculaire au plan de l'ouverture et qui passe par son barycentre.

L'épaisseur du dispositif ou d'une branche est mesurée suivant la direction de l'axe X.

La longueur et la hauteur de l'ouverture, du dispositif ou d'une branche sont mesurées dans un plan transversal perpendiculaire à la direction de l'axe X, la direction de la hauteur étant perpendiculaire à la direction de la longueur.

Sauf indication contraire, la hauteur et l'épaisseur d'un organe désignent la hauteur maximale et l'épaisseur maximale en considérant l'ensemble des plans perpendiculaires à l'axe principal de l'organe, respectivement.

Par « pincement », on entend une action exercée sur les jonctions droite et gauche pour les rapprocher l'une de l'autre, et ainsi élargir l'ouverture. Avantageusement, cette action peut être exercée par pression du poucet et de l'index d'une même main de l'utilisateur sur lesdites jonctions.

Une branche est « pliable » lorsqu'elle s'incurve progressivement sous l'effet d'un rapprochement de ses extrémités. Cette courbure permet d'obtenir, dans la position de montage une ouverture délimitée par un bord concave sensiblement sur toute la longueur des branches. La forme générale de l'ouverture évolue progressivement depuis une forme de fente ou fusiforme vers la forme d'un disque, à mesure du pincement.

Le « bord d'appui » d'une branche est la surface de la branche qui est destinée à compresser le pénis de l'utilisateur dans la position de service.

Par « monobloc », on entend que le dispositif n'est pas constitué en plusieurs pièces.

La circularité d'une section ou d'une ouverture est le rapport de la plus petite dimension de la section ou de l'ouverture sur la plus grande dimension de la section ou de l'ouverture, respectivement. Un disque a par exemple une circularité de 1.

« Comporter » ou « comprendre » ou « présenter » doivent être interprétés de manière non limitative.

### Description détaillée

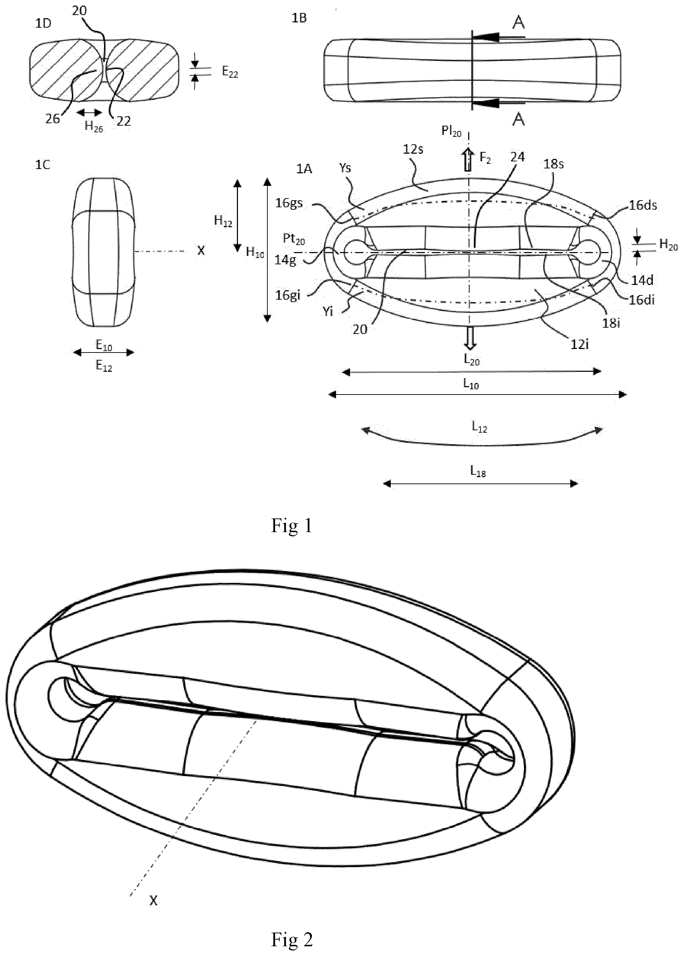

La figure 1 représente, dans la position de repos, un dispositif 10 selon un mode de réalisation préféré de l'invention.

De préférence, le dispositif est monobloc. Avantageusement, il est rapide, fiable et peu coûteux à fabriquer. De préférence, il est fabriqué par moulage.

La surface extérieure du dispositif est de préférence lisse, de préférence dépourvue d'arête vive et de préférence dépourvue de cavité, ce qui limite l'accumulation de saleté et donc améliore l'hygiène.

De préférence, le dispositif ne présente pas de zone en contre-dépouille incompatible avec une fabrication par moulage.

Plus de 80%, plus de 90%, plus de 95% de la surface extérieure du dispositif est de préférence convexe. Dans un mode de réalisation, la surface extérieure est convexe en tout point, hormis dans la région des jonctions droite et gauche.

La surface extérieure du dispositif qui délimite l'ouverture est de préférence en un matériau présentant un coefficient de frottement suffisant pour le maintenir dans la position de service lorsque l'utilisateur le porte.

Le dispositif comporte des branches supérieure 12s et inférieure 12i, d'axes principaux Ys et Yi, respectivement, et des jonctions droite 14d et gauche 14g reliant respectivement des extrémités droites 16ds et 16di des branches supérieure et inférieure respectivement, et des extrémités gauches 16gs et 16gi des branches supérieure et inférieure respectivement.

De préférence, la longueur de chaque branche, mesurée selon son axe principal, est supérieure à 1,8 fois, de préférence supérieure à 1,9 fois, de préférence supérieure à deux fois, et/ou inférieure à 3 fois, de préférence inférieure à 2,5 fois, de préférence inférieure à 2,3 fois, de préférence inférieure à 2,2 fois, la plus grande dimension transversale, c'est-à-dire mesurée dans une section transversale, en considérant toutes les sections transversales le long de la branche. La section transversale est de préférence variable le long de l'axe principal.

Les branches supérieure 12s et inférieure 12i comportent des bords d'appui 18s et 18i, respectivement, qui délimitent une ouverture 20.

De préférence, une branche, de préférence chaque branche, et/ou une jonction, de préférence chaque jonction, de préférence le dispositif est constitué(e) en un unique matériau, de préférence un élastomère, de préférence un silicone, de préférence un silicone choisi pour être non allergisant au contact de la peau. Le silicone est de préférence un silicone en polyaddition.

De préférence, une jonction, de préférence chaque jonction, est dans le même matériau que les branches.

De préférence, un matériau, de préférence chaque matériau d'une branche, de préférence de chaque branche, et/ou d'une jonction, de préférence de chaque jonction, de préférence du dispositif présente :
- une dureté Shore A supérieure à 20, de préférence supérieure à 25, de préférence supérieure à 30, et/ou inférieure à 40, de préférence inférieure à 35 ; et/ou
- une résistance à la traction supérieure à 3 N/mm², de préférence supérieure à 4 N/mm², de préférence supérieure à 5 N/mm², et/ou inférieure à 10 N/mm², inférieure à 8 N/mm² ; et/ou
- est translucide, pour en améliorer la discrétion.

L'élasticité est de préférence suffisante pour que le dispositif reprenne sa position de repos après utilisation.

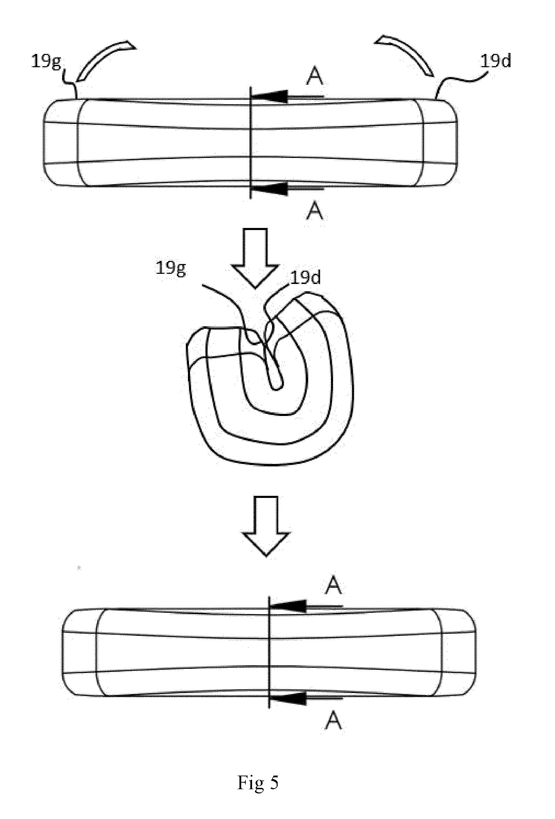

Comme illustré sur la figure 5, le dispositif est de préférence constitué en un ou plusieurs matériaux suffisamment souple(s) et élastique(s) pour que, à partir de la position de repos, deux points 19d et 19g à la périphérie du dispositif (ce qui exclut les points de la surface qui définit l'ouverture 20) et aux extrémités droite et gauche du dispositif, respectivement, puissent être rapprochés jusqu'à se toucher (figure 5), sans déformation plastique ni rupture du dispositif. Autrement dit, lorsque l'effort de rapprochement cesse, du fait de son élasticité, le dispositif reprend la position de repos de la figure 1B.

De préférence, une branche, de préférence chaque branche, est pleine.

De préférence, une branche, de préférence chaque branche, est extérieurement bombée, comme illustré sur les figures.

### Position de repos

De manière générale, le dispositif présente la forme d'un grain de riz aplati. Vu de face, le contour extérieur du dispositif est ovale ou ellipsoïdal. Le contour intérieur délimite une ouverture sous la forme d'une fente prolongée à chacune de ses extrémités par un orifice traversant, sensiblement cylindrique de base circulaire, en communication avec la fente.

De préférence, dans la position de repos,
- la longueur L₁₀ du dispositif est de préférence supérieure à 50 mm, ou supérieure à 60 mm, et/ou inférieure à 80 mm ; et/ou
- la hauteur H₁₀ du dispositif est de préférence supérieure à 30 mm et/ou inférieure à 40 mm; et/ou
- l'épaisseur E₁₀ du dispositif est de préférence supérieure à 12 mm, de préférence supérieure à 15 mm, et/ou inférieure à 18 mm ; et/ou
- la longueur L₁₂ d'au moins une branche, de préférence de chaque branche, est de préférence supérieure à 55 mm, de préférence supérieure à 60 mm, et/ou inférieure à 75 mm, de préférence inférieure à 70 mm ; et/ou
- la hauteur H₁₂ d'au moins une branche, de préférence de chaque branche, est de préférence supérieure à 12 mm, de préférence supérieure à 15 mm et/ou inférieure à 20 mm; et/ou
- l'épaisseur E₁₂ d'au moins une branche, de préférence de chaque branche, est de préférence supérieure à 12 mm, de préférence supérieure à 15 mm, et/ou inférieure à 18 mm; et/ou
- la hauteur H₁₂ et/ou l'épaisseur E₁₂ d'au moins une branche, de préférence de chaque branche, est maximale à mi-longueur de la branche ; et/ou
- une branche, de préférence chaque branche, présente, sur un segment de la branche représentant plus de 80%, de préférence plus de 90%, de préférence 100%, de la longueur L₁₂ de la branche, un profil transversal, c'est-à-dire dans une coupe transversale (voir figure 1D), présentant une circularité supérieure à 0,6, supérieure à 0,7 ou supérieure à 0,8 ; et/ou
- une branche, de préférence chaque branche, présente, sur un segment de la branche représentant plus de 80%, de préférence plus de 90%, de préférence 100% de la longueur L₁₂ de la branche, un profil transversal présentant une aire de préférence supérieure à 200 mm², de préférence supérieure à 220 mm², et/ou inférieure à 300 mm², de préférence inférieure à 250 mm² ; et/ou
- la partie de ladite section transversale définissant un bord d'appui d'une branche, de préférence de chaque branche présente un méplat 22, l'épaisseur E₂₂ du méplat, mesurée suivant la direction de l'épaisseur du dispositif, étant de préférence supérieure à 0,5 mm et/ou inférieure à 2 mm, de préférence inférieure à 1 mm ; et/ou
- la partie de ladite section transversale définissant un bord d'appui présente une forme arrondie, ou « bombée », la hauteur H₂₆ du bombement 26 étant de préférence supérieure à 3 mm et/ou inférieure à 10 mm ;
- ladite section transversale présente une forme générale rectangulaire, de préférence à coins arrondis, le bord du côté de l'ouverture étant bombé, comme illustré sur la figure 1D, la hauteur H₂₆ du bombement 26 étant de préférence supérieure à 3 mm et/ou inférieure à 10 mm ; et/ou
- ladite section transversale présente un profil s'effilant radialement vers l'extérieur, c'est-à-dire dont la hauteur diminue à mesure que l'on s'écarte de l'ouverture, vers l'extérieur, comme illustré sur lafigure 1D ; et/ou
- la longueur L₁₈ du bord d'appui d'une branche, de préférence de chaque branche, mesurée suivant la direction de la longueur du dispositif, représente plus de 70%, de préférence plus de 80%, voire plus de 90%, de la longueur L₁₀ du dispositif ; et/ou
- le bord d'appui d'au moins une branche, de préférence de chaque branche délimitant l'ouverture présente une longueur L₁₈ supérieure à 40 mm et/ou inférieure à 65 mm, de préférence inférieure à 60 mm, de préférence inférieure à 50 mm ; et/ou
- le bord d'appui supérieur de la branche supérieure s'étend sensiblement parallèlement au bord d'appui inférieur à la branche inférieure ; et/ou
- le bord d'appui d'au moins une branche, de préférence de chaque branche délimitant l'ouverture est sensiblement rectiligne, le bord d'appui présentant de préférence un bombement central 24, la hauteur du bombement central étant de préférence inférieure à 2 mm, de préférence inférieure à 1 mm et/ou supérieure à 0,3 mm ; et/ou
- la longueur L₂₀ de l'ouverture représente plus de 90%, de préférence plus de 95%, de préférence plus de 98% de la longueur L₁₂ du dispositif ; et/ou
- la hauteur H₂₀ de l'ouverture représente moins de 20%, de préférence moins de 10% de la hauteur H₁₀ du dispositif ; et/ou
- la hauteur H₂₀ de l'ouverture dans le segment délimité par les branches, de préférence à mi-longueur de l'ouverture, est de préférence supérieure à 0,1 mm , de préférence supérieure à 0,5 mm et/ou inférieure à 15 mm, de préférence inférieure à 10 mm, de préférence inférieure à 5 mm, et/ou
- la longueur L₂₀ de l'ouverture dans le segment délimité par les branches, de préférence à mi-hauteur de l'ouverture, est de préférence supérieure à 40 mm et/ou inférieure à 70 mm, et/ou
- l'ouverture est symétrique par rapport à un plan longitudinal médian Pl₂₀ et de préférence par rapport à un plan transversal médian Pt₂₀ ; et/ou
- le dispositif est symétrique par rapport au plan longitudinal médian Pl₂₀ et de préférence par rapport à un plan transversal médian Pt₂₀ ; et/ou
- l'ouverture s'étend dans un plan.

Une ouverture étroite en hauteur, sous la forme d'une fente, permet avantageusement de maximiser la hauteur et l'épaisseur des branches, avec un encombrement limité.

Une jonction, de préférence chaque jonction est de préférence venue de matière avec les branches.

Une jonction, de préférence chaque jonction, présente de préférence la forme d'un C ouvrant vers l'ouverture 20, comme illustré sur la figure 1A.

De préférence, le bord de la jonction qui délimite l'ouverture 20 est en forme de C, la hauteur de ce C étant de préférence supérieure à l'écartement entre les deux extrémités des branches connectées à la jonction, de préférence supérieure à deux fois ou à trois fois cet écartement, comme représenté sur la figure 1A.

Le rayon de courbure du bord de la jonction en forme de C est de préférence supérieur à 1 mm, de préférence supérieur à 2 mm, de préférence supérieur à 3 mm et/ou inférieur à 20 mm, de préférence inférieur à 15 mm, de préférence inférieur à 10 mm, de préférence inférieur à 8 mm, de préférence inférieur à 6 mm, de préférence inférieur à 5 mm en tout point quelconque de ce bord.

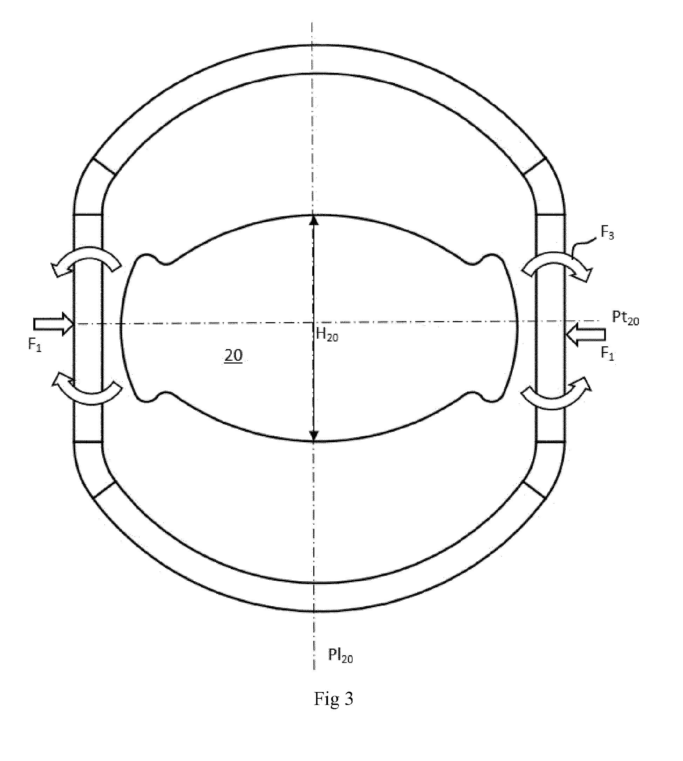

Les extrémités des branches connectées à une jonction sont dans le prolongement du C, de sorte que l'aplatissement du C sous l'effet du pincement (flèches F₃ sur la figure 3) conduit à un écartement des deux branches l'une de l'autre, selon la direction de la hauteur. Il est ainsi avantageusement possible d'augmenter de l'aire de l'ouverture depuis la position de repos, avec un dispositif particulièrement peu encombrant.

L'écartement des deux branches l'une de l'autre, selon la direction de la hauteur, illustré par les flèches F₂ sur la figure 1, par aplatissement des jonctions (flèches F₃ sur la figure 3), permet un élargissement de l'ouverture dans une première phase du pincement, avec une flexion très faible, voire nulle des branches. L'intensité de l'effort de pincement nécessaire pour aplatir les jonctions dépend de l'épaisseur de matière qui les constitue.

Dans une deuxième phase du pincement, l'ouverture poursuit son développement principalement du fait que les branches se plient, ce qui arrondit l'ouverture.

L'effort de pincement peut être différent dans la première phase et dans la deuxième phase. Il est de préférence plus faible dans la première phase.

De préférence, une jonction, de préférence chaque jonction, est en un matériau élastique. Le pliement des branches produit alors un étirement élastique des jonctions. Cet étirement conduit à un écartement supplémentaire des deux branches l'une de l'autre, selon la direction de la hauteur, et contribue encore à l'élargissement de l'ouverture, c'est-à-dire à l'augmentation de son aire.

La forme en C et la constitution en un matériau élastique permettent ainsi d'augmenter considérablement de l'aire de l'ouverture 20 par écartement des branches au niveau des jonctions. Même si ce n'est pas le mode de réalisation préféré, un tel élargissement de l'ouverture est possible même si les branches sont peu flexibles.

Des essais ont également montré que de telles jonctions en C, en un matériau élastique, augmente considérablement la durée de vie du dispositif, qui peut dépasser une année. En particulier, elles évitent l'apparition rapide de fissures constatées avec des jonctions en V.

### Position de montage

La position de montage correspond à la configuration que le dispositif adopte provisoirement pour être disposé sur le pénis de l'utilisateur. De préférence, l'aire de l'ouverture 20 est alors de préférence sensiblement maximale.

Les dimensions des branches (longueur, hauteur, épaisseur) sont sensiblement les mêmes dans la position de montage et dans la position de repos. L'épaisseur du dispositif est également sensiblement la même dans la position de montage et dans la position de repos.

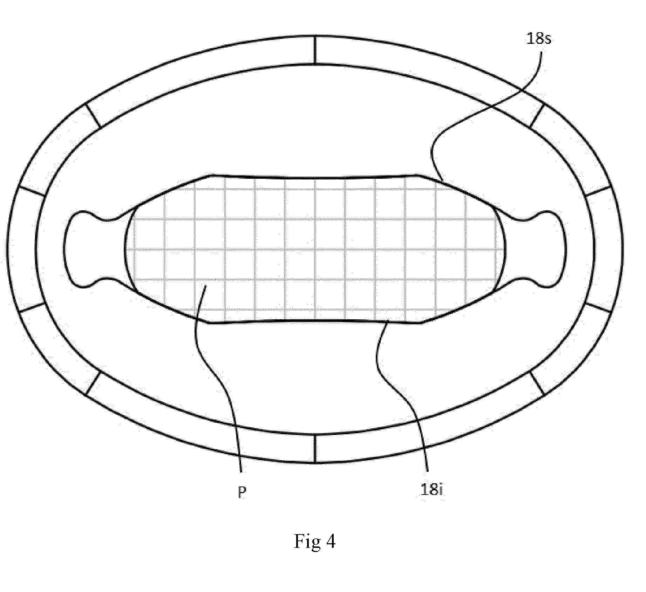

De préférence, dans la position de montage,
- la longueur L₁₀ du dispositif est de préférence supérieure à 40 mm et/ou inférieure à 60 mm; et/ou
- la hauteur H₁₀ du dispositif est de préférence supérieure à 40 mm et/ou inférieure à 60 mm; et/ou
- les bords d'appui des branches sont courbées, les centres de courbure étant du côté de l'ouverture ; et/ou
- la hauteur H₂₀ de l'ouverture, de préférence à mi-longueur de l'ouverture, est de préférence supérieure à 30 mm et/ou inférieure à 50 mm, et/ou
- la longueur L₂₀ de l'ouverture, de préférence à mi-hauteur de l'ouverture, est de préférence supérieure à 23 mm et/ou inférieure à 50 mm, et/ou
- le rapport de la longueur L₂₀ de l'ouverture sur la hauteur H₂₀ de l'ouverture est supérieur à 0,8 et/ou inférieur à 1,2 ; et/ou
- le rapport de la hauteur H₂₀ de l'ouverture dans la position de montage (figure 3) sur la hauteur H₂₀ de l'ouverture dans la position de repos (figure 1 ou figure 2) est supérieur à 5, de préférence supérieur à 10 et/ou inférieur à 100 ;
- le rapport de la hauteur H₂₀ de l'ouverture dans la position de montage (figure 3) sur la hauteur H₂₀ de l'ouverture dans la position de service (figure 4) est supérieur à 1,5, de préférence supérieur à 2 et/ou inférieur à 4, de préférence inférieur à 3 ;
- l'ouverture est symétrique par rapport au plan longitudinal médian Pl₂₀ et de préférence par rapport au plan transversal médian Pt₂₀ ; et/ou
- le dispositif est symétrique par rapport au plan longitudinal médian Pl₂₀ et de préférence par rapport à un plan transversal médian Pt₂₀ ; et/ou
- l'ouverture s'étend dans un plan.

### Position de service

La forme du dispositif dans la position de service dépend de la morphologie de l'utilisateur.

L'aire de l'ouverture est intermédiaire entre l'aire de l'ouverture dans la position de repos et l'aire de l'ouverture dans la position de montage.

### Fonctionnement

Le fonctionnement du dispositif découle directement de la description qui précède.

Pour passer de la position de repos (figures 1 et 2) à la position de montage (figure 3), l'utilisateur pince latéralement le dispositif, de préférence avec une seule main. Sous l'effet de ce pincement, illustré par les flèches F₁ de la figure 3, les jonctions s'aplatissent et les branches se courbent vers l'extérieur, élargissant ainsi l'ouverture 20. Les jonctions en C permettent de bien contrôler l'élargissement de l'ouverture. La symétrie du dispositif permet avantageusement de le disposer avec la branche supérieure au-dessus ou en dessous de la branche inférieure, sans modification de l'efficacité du dispositif. Le dispositif est avantageusement particulièrement simple à utiliser.

Le dispositif est de préférence configuré de manière que, lors du pincement, un effort de compression de 5 N permette une augmentation de la hauteur de l'ouverture de plus de 20 mm.

De préférence, les branches ne comportent pas d'âme de rigidification. Lors du pincement, elles peuvent donc vriller sur elles-mêmes. Si le pincement n'est pas réalisé correctement, le dispositif peut également « flamber », c'est-à-dire plier autour d'un axe horizontal parallèle au plan général du dispositif. La section transversale, et en particulier l'épaisseur des branches, limitent avantageusement ce risque.

De préférence, le contour de l'ouverture 20 prend une forme sensiblement arrondie dans la position de montage, au moins le long des branches, et de préférence une forme sensiblement rectiligne au niveau des jonctions, comme illustré sur la figure 3. Le dispositif est donc très peu encombrant.

L'utilisateur peut ensuite disposer très facilement le dispositif sur pénis P, puis le relâcher. Le dispositif se referme alors élastiquement, partiellement, jusqu'à la position de service (voir figure 4). Aucune autre opération n'est avantageusement requise.

Dans la position de service, les bords d'appui des branches sont en appui sur le pénis de préférence sur plus de 80%, de préférence plus de 90%, de préférence sensiblement 100% de leur longueur.

La pression exercée sur l'urètre résulte de l'élasticité des branches, mais aussi de l'élasticité des jonctions.

La souplesse du matériau utilisé permet au dispositif de prendre la forme des parties du corps sur lesquelles il prend appui, ce qui le rend discret. La surface d'appui augmente avec l'aire de l'ouverture, ce qui limite les risques de lésion et améliore le confort, par un effet « coussin ». L'appui est cependant confortable et efficace, des essais ayant montré que l'incontinence est supprimée.

Le rapprochement des jonctions l'une de l'autre permet par ailleurs de repousser de la matière vers le milieu des branches. Des plis peuvent en résulter au milieu des bords d'appui, ce qui, avantageusement permet de créer une protubérance centrale et d'augmenter localement la pression au niveau de l'urètre.

Pour uriner, l'utilisateur peut pincer à nouveau le dispositif afin de supprimer la pression sur l'urètre, de préférence avec une seule main. Il n'est pas nécessaire de retirer le dispositif. Lorsque l'utilisateur cesse de pincer le dispositif, ce dernier revient élastiquement dans la position de service.

Pour retirer le dispositif, l'utilisateur peut pincer à nouveau le dispositif pour revenir à la position de montage, ou à une configuration similaire, puis le glisser sur le pénis pour l'en détacher. Le dispositif peut être alors facilement nettoyé, manuellement ou à la machine. L'absence de cavité à la surface du dispositif facilite le séchage et limite le risque d'accumulation de saleté.

Le dispositif peut être réutilisé.

Comme cela apparaît clairement à présent, un dispositif selon l'invention présente, dans le mode de réalisation préféré, les avantages suivants :
- il peut être mis en position de service ou être retiré avec une seule main et facilement, par un simple pincement ;
- il est monobloc, ce qui le rend robuste, mais aussi peu coûteux à fabriquer ;
- il est en un matériau mou, ce qui permet l'application d'une pression élevée sur l'urètre, sans traumatisme, et confère des propriétés de déformation avantageuses pour épouser le corps de l'utilisateur dans la position de service ;
- il présente des branches suffisamment épaisses pour s'incurver progressivement vers l'extérieur à mesure du pincement, sans flambage ;
- il présente des branches suffisamment molles pour qu'elles puissent être fortement courbées, sans se rompre ou se fatiguer. Avec le faible encombrement du dispositif, cette déformabilité contribue à rendre le dispositif particulièrement discret dans la position de service.

Bien entendu, l'invention n'est pas limitée au mode de réalisation décrit et représenté.

## Revendications

1. Dispositif de compression pénienne monobloc comportant :
- des branches supérieure (12s) et inférieure (12i), et
- des jonctions droite (14d) et gauche (14g) reliant des extrémités droites (16ds, 16di) des branches supérieure et inférieure, et des extrémités gauches (16gs, 16gi) des branches supérieure et inférieure, respectivement, de manière à délimiter une ouverture (20),
au moins une des branches supérieure et inférieure, dite « branche pliable », étant pliable élastiquement par pincement du dispositif entre les jonctions droite et gauche, dispositif dans lequel ladite branche pliable est constituée en un ou plusieurs matériaux présentant une dureté Shore A inférieure à 40.

2. Dispositif selon la revendication immédiatement précédente, dans lequel ladite branche pliable présente, sur un segment de ladite branche pliable représentant plus de 80% de la longueur (L₁₂) de ladite branche pliable, un profil transversal présentant une aire supérieure à 150 mm².

3. Dispositif selon l'une quelconque des revendications précédentes, dans lequel au moins une des jonctions droite et gauche, de préférence chaque jonction droite et gauche est déformable élastiquement sous l'effet du pincement de manière à écarter les extrémités de la branche supérieure et de la branche inférieure qui sont physiquement connectées à ladite jonction.

4. Dispositif selon la revendication immédiatement précédente, dans lequel chaque dite jonction déformable élastiquement présente la forme d'un C débouchant vers l'ouverture, les branches étant reliées aux extrémités du C, le rayon de courbure du bord de chaque jonction en forme de C étant supérieur à 1 mm et inférieur à 20 mm en tout point dudit bord, de manière que les branches s'écartent l'une de l'autre par aplatissement du C de chaque dite jonction sous l'effet d'un pincement.

5. Dispositif selon la revendication immédiatement précédente, le rayon de courbure du bord de chaque jonction en forme de C étant supérieur à 2 mm et inférieur à 6 mm en tout point dudit bord.

6. Dispositif selon l'une quelconque des revendications précédentes, configuré pour s'ouvrir, par pincement, d'abord principalement par aplatissement de chaque jonction, puis principalement par pliement des branches.

7. Dispositif selon l'une quelconque des revendications précédentes, le dispositif étant constitué en un ou plusieurs élastomères et étant fabriqué par moulage.

8. Dispositif selon l'une quelconque des revendications précédentes, les branches ne comportant pas d'âme de rigidification, le dispositif étant de préférence constitué en silicone.

9. Dispositif selon l'une quelconque des revendications précédentes, dont plus de 90% la surface extérieure est convexe.

10. Dispositif selon l'une quelconque des revendications précédentes, configuré de manière que le rapport de la hauteur de l'ouverture dans la position de montage sur la hauteur de l'ouverture dans la position de repos est supérieur à 5.

11. Dispositif selon l'une quelconque des revendications précédentes, ladite ouverture étant telle que, dans une position de montage, le rapport de la longueur (L₂₀) de l'ouverture sur la hauteur (H₂₀) de l'ouverture est supérieur à 0,8.

12. Dispositif selon l'une quelconque des revendications précédentes, configuré de manière que l'ouverture présente la forme d'une fente dans la position de repos, et présente un contour arrondi le long des branches et un contour sensiblement rectiligne le long des jonctions dans une position de montage obtenue, à partir de la position de repos, par pincement du dispositif entre les jonctions droite et gauche.

13. Dispositif selon l'une quelconque des revendications précédentes, dans lequel l'ouverture (20) est symétrique par rapport à un plan longitudinal médian (Pl₂₀) et par rapport à un plan transversal médian Pt₂₀.

14. Dispositif selon l'une quelconque des revendications précédentes, constitué en un ou plusieurs matériaux suffisamment souple(s) et élastique(s) pour que, à partir de la position de repos, deux points (19d, 19g) à proximité des extrémités droite et gauche du dispositif, respectivement, puissent être rapprochés jusqu'à se toucher, sans déformation plastique ni rupture du dispositif.

15. Dispositif selon l'une quelconque des revendications précédentes, présentant, vu de face, un contour extérieur ovale ou ellipsoïdal, et un contour intérieur délimitant une ouverture sous la forme d'une fente, de préférence prolongée à chacune de ses extrémités par un orifice traversant, sensiblement cylindrique de base circulaire, en communication avec la fente.
